# EUROPEAN PATENT APPLICATION

(11) **EP 3 992 613 A1**
(43) Date of publication of application: **04.05.2022**
(21) Application number: 20204368.3
(22) Date of filing: 28.10.2020
(51) Int. Cl.: G01N 21/25, G01N 21/31, G01N 21/78, G01N 33/48

(54) **SPUTUM ANALYSIS METHOD AND SYSTEM**

(71) Applicant: Koninklijke Philips N.V., 5656 AG Eindhoven (NL)
(72) Inventor: BRANS, Harold Johannes Antonius, 5656 AE Eindhoven (NL); DELLIMORE, Kiran Hamilton J., 5656 AE Eindhoven (NL)
(74) Representative: Philips Intellectual Property & Standards

(57) **Abstract**

A method (20) is for analysis of sputum samples (34) of a subject, for use in monitoring respiratory health of the subject. The method comprises acquiring optical sensing data of a sputum sample at a plurality of spatial locations (44) across the sample, and determining a respective color measurement for each of the spatial locations. The method further comprises determining a property associated with the sample, or determining health information related to the patient, based on the combination of color measurements from the plurality of locations. More detailed and precise analysis of the sputum can be achieved compared to acquiring a single color measurement, which allows for more precise monitoring of the patient progress. Additional information not attainable with a single color measurement can also be derived.

## Description

### FIELD OF THE INVENTION

This invention relates to a method and system for analyzing sputum samples of a subject.

### BACKGROUND OF THE INVENTION

Many patients with chronic respiratory diseases, such as chronic obstructive pulmonary disease (COPD), cystic fibrosis (CF) and non CF-bronchiectasis, experience severe mucus (or sputum) build up in their airway system. Consequently, clearing the airways of mucus becomes more difficult. This will lead to accumulation of bacterial load, which leads to disease exacerbations.

The purulence of sputum is known to be associated with this bacterial load in patients with exacerbations. In order to standardize the evaluation of the purulence of sputum, the Sputum Color Chart was developed (see for example: "Sputum color, a useful clinical tool for non CF Bronchiectasis", Murray et al, Eur Respi J 2009, 34: 361-364).

Using this color chart, a sputum sample can be matched with the closest color on the chart. The number of colors on the chart can vary from at least 3, to 7 or more.

Each color on the chart relates to particular health state of the patient, e.g. a particular exacerbation condition. Interpretation and application of the Color Chart is operator dependent and experience is needed. This can lead to inconsistency between measurements, and experienced clinicians are required to perform the analysis. As a result, at home monitoring is currently not possible.

An underlying reason for the irreproducibility of measurements is the lack of color homogeneity of most sputum samples. An objective judgement on the average color is difficult, even for highly experienced clinicians.

The use of a color chart also allows for only low resolution measurement, since it is composed of only a limited number of colors. This resolution is not great enough for detailed analysis. Detailed monitoring would be useful to track sputum removal efficacy and therapy effectiveness. If removal of mucus in the airways is too inefficient, bacterial load will increase. This change in bacterial load results in a change in color. The effectiveness of a removal therapy can therefore be correlated to a decrease in bacterial load, by looking at the color of sputum.

With the color composition of sputum samples, it is also possible to distinguish between different exacerbations or medical conditions. Table 1 below for example shows how different color contents of sputum are associated with different medical conditions.

**Table 1**

| | green or yellow | brown | white | black | clear | red or pink |
|---|---|---|---|---|---|---|
| allergic rhinitis | | | | | ✔ | |
| bronchitis | ✔ | ✔ | ✔ | | ✔ | |
| COPD | | | ✔ | | | |
| congestive heart failure | | | ✔ | | | ✔ |
| cystic fibrosis | ✔ | ✔ | | | | |
| fungal infection | | | | ✔ | | |
| gastroesophageal reflux disease (GERD) | | | ✔ | | | |
| lung abscess | | ✔ | | ✔ | | ✔ |
| lung cancer | | | | | | ✔ |
| pneumonia | ✔ | ✔ | | | ✔ | ✔ |
| pneumoconiosis | | ✔ | | ✔ | | |
| pulmonary embolism | | | | | | ✔ |
| sinusitis | ✔ | | | | | |
| smoking | | | | ✔ | | |
| tuberculosis | | | | | | ✔ |

Improvements in the analysis of sputum samples, able to overcome one or more of the problems outlined above, would be of value.

### SUMMARY OF THE INVENTION

The invention is defined by the claims.

According to examples in accordance with an aspect of the invention, there is provided a sputum analysis method for analyzing a sputum sample of a subject. The method comprises receiving from an optical sensing device optical sensing data relating to a plurality of spatial locations of the sputum sample. The method further comprises determining based on the sensing data a respective sputum color measurement for each of the plurality of spatial locations. The method further comprises applying an analysis operation comprising determining one or more properties of the sputum sample and/or one or more health parameters of the subject based on the color measurements for the plurality of spatial locations. The method further comprises generating a data output based on the result of the analysis operation.

Embodiments of the present invention are based on obtaining a digital measurement of the sputum color at multiple locations and deriving a further property of the sputum, or a health-related information about the subject based thereon. Thus, embodiments provide at least three advantages over the standard color-chart method for assessing sputum. First, the color measurements are made more objective and more precise since they are performed based on optical measurement data, rather than by sight of a human operator. Second, the method provides for computationally deriving a further relevant parameter from the color measurements, which adds objectivity to the analysis applied to the sputum color. For example, the parameter could be a measure of disease progression, or could be simply a physical property of the sputum (e.g. color distribution, or estimated bacterial load) which can be used as a proxy for a health indicator. Third, by acquiring color measurements for a plurality of different spatial locations, greater resolution is provided to the color assessment compared to standard methods which consider the whole sample to have a single 'average' color. In embodiments of this invention, variation in color across the sample can be taken into account, and small amounts of certain particularly significant colors (e.g. red for blood) can be detected even if in only small areas of the sample.

The optical sensing data should include data representative of the true (original) color of the sputum sample.

The one or more properties of the sputum may by way of example include: color homogeneity, spatial color distribution, color composition (e.g. list of all colors present), color spread (e.g. number of colors present). The one or more properties may additionally or alternatively include medically relevant properties such as bacterial load, or binary properties indicative of presence or absence of one or more pre-defined warning colors, e.g. red for blood.

In accordance with one or more embodiments, the analysis operation may comprise determining a measure of color homogeneity of the sample, based on the color measurements for the plurality of spatial locations.

In accordance with one or more embodiments, the analysis operation may comprise detecting presence of any of a set of pre-defined reference colors at any of the spatial locations. One of the sputum properties derived in the analysis operation may be a binary property indicative of presence or absence of any of said reference colors.

The reference colors could be indicative of danger signs, for example blood presence. In standard assessment methods, the sputum color is spatially averaged over a whole area of the sample, meaning that early presence of red blood is not detected.

Additionally or alternatively, the analysis operation may comprise determining one or more health parameters, properties or information relating to the subject. These may include for example a medical condition of the subject, a severity of a medical condition, and/or an indicator of sputum removal efficacy.

In accordance with one or more embodiments, the method may further comprise comparing results of the analysis operation with a source of historical sputum analysis information for the subject, and detecting change over time of one or more of the sputum properties or subject health parameters. For example, a trend in the one or more properties or parameters over a plurality of measurement events may be derived.

In accordance with one or more embodiments, the optical sensing data for the sample may include sensing data for a series of time points spanning a measurement period. The color measurements may be derived for the plurality of spatial locations for each of the series of time points. This thereby obtains dynamic color measurement information for the sample. The dynamic color information allows additional properties of the sputum to be derived, such as viscosity or stability.

In one set of examples, the analysis operation may comprise determining a measure indicative of changes in a spatial color distribution across the sample over the measurement period.

Additionally or alternatively, in accordance with one or more examples, the analysis operation may comprise identifying one or more physical features within the sample based on the sensing data, and tracking movement of features over the measurement period.

The movement may be due to Brownian motion and/or due to an external pressure flow exerted manually. By monitoring this, rheological (flow) behavior of the sample can be derived, and/or a movement speed of the features.

The analysis operation may comprise comparing the plurality of color measurements, or a property derived therefrom, with a source of reference information, to thereby derive the one or more properties of the sputum sample or the one or more health parameters. The reference information may for example be a lookup table stored in a local or remote datastore. The lookup table may associate particular spatial distribution of colors with a particular value of a property.

Examples in accordance with a further aspect of the invention provide a computer program product comprising computer program code configured, when run on a processor, to cause the processor to perform a method in accordance with any example or embodiment outlined above or described below, or in accordance with any claim of this application.

Examples in accordance with a further aspect of the invention may also provide a system for acquiring the optical sensing data of the sample and executing the analysis method based on that data.

The sputum analysis system according to one or more embodiments comprises a measurement device, wherein the measurement device comprises: a housing, and an optical sensing device fixed relative to the housing and operable to emit optical signals into a sensing region and acquire optical sensing data based thereon, for thereby acquiring optical sensing data of a sputum sample received in the sensing region. The system further comprises a processing arrangement adapted to receive optical sensing data from the optical sensing device and perform an analysis method in accordance with any example or embodiment outlined above or described below, or in accordance with any claim of this application.

In one or more sets of embodiments, the measurement device may further include a receptacle receiving section arranged to receive a removable sputum sample receptacle. It may further be adapted to hold the receptacle within said sensing region during operation for optical sensing of a sputum sample in the receptacle. Alternatively, in some examples, the sputum sample may be transferred to a separate measurement chamber, and wherein the separate measurement chamber is positioned within said sensing region.

In this example, the measurement device is adapted to hold the sample receptacle in a defined spatial relationship (preferably static) relative to the optical sensing device. This provides for more reliable and reproducible results. The measurement device may in this case be a tabletop measurement device for instance. However, this is not essential.

For example, according to a further set of embodiments, the measurement device may take the form of a handheld unit for performing optical measurement. The handheld unit could be a dedicated handheld sensing device, e.g. with integrated spectrophotometer, or could be facilitated by a mobile computing device, and wherein an integrated camera of the mobile computing device is used as the optical sensing device.

Furthermore, there are different options for the location of the processing arrangement. In at least one set of embodiments, the processing arrangement may be integrated in the measurement device. Here, the system provides an integrated measurement device which is adapted to perform the processing operations locally to analyze the sample.

In a further set of embodiments, the processing arrangement may be comprised externally to the measurement device, e.g. by a mobile computing device, and adapted to establish a communication channel with the measurement device during operation to receive the optical sensing data. In this case, the system is a distributed system, wherein the measurement device acquires the measurement data for the sample, but the analysis is performed externally. For example, an app installed on a smartphone may be used to derive the color measurement information, and apply the analysis operation. The two devices may be connected by a direct communication channel, or may be connected indirectly, for example via an internet link, or through a cloud based interface.

In accordance with one or more embodiments, the measurement device comprises a control module adapted to execute a pre-defined measurement protocol, the measurement protocol comprising: receiving a signal indicative of receipt of the sputum sample in the receiving section; activating a sample conditioning procedure responsive to receipt of the signal, for adjusting one or more physical properties of the sample; and subsequently activating an optical sensing procedure to acquire the optical sensing data of the sample.

This defines execution of an ordered control program.

In advantageous examples, the conditioning procedure does not change a color of at least one region of the sample and the optical sensing data are acquired for said at least one region. This is so that the optical sensing data can be representative of the true color of the sputum sample.

The control module is local to the measurement device. It may be the same as the processing arrangement or different.

The function of the conditioning process is to ensure reliable and consistent measurement conditions at each measurement event.

In accordance with one or more embodiments, the conditioning procedure may comprise adjusting a temperature of the sample to within a pre-defined temperature range based on use of one or more heating elements comprised by the measurement device. The temperature adjustment can be performed to ensure ensures a consistent viscosity. The measurement device may comprise one or more heating elements arranged to be in thermal communication with the sample receptacle and/or sample contents when the receptacle is received in the receiving section.

Additionally, or alternatively, the conditioning procedure may comprise adding a sample conditioning agent into the sample. It preferably further comprises mixing the conditioning agent into the sample. The conditioning agent may be an agent which has the property of altering a consistency or viscosity of the sputum sample. For example it may include a saline solution. The conditioning agent does not change a color of the sample.

It is preferable that the sputum receptacle is a disposable receptacle, with pre-defined dimensions and internal volume. It preferably includes markings to indicate a target fill level to the user. It is preferably made of optical grade plastic. One aspect of the invention provides a kit of parts comprising a measurement system according to any of the embodiments described in this disclosure, in combination with at least one sputum sample receptacle with one or more of the features mentioned above.

In accordance with one or more embodiments, the measurement protocol may further comprise: adding a reagent to at least one region of the sample, the reagent adapted to stimulate a color-change response dependent upon an analyte content in the sample; and acquiring optical sensing data of said at least region, for use in detecting the color-change response.

The optical sensing operation should comprise acquiring optical sensing data of the reagent color change response in addition to detecting the true, original color of the sputum sample. This can be achieved in different ways. One way is to use a sample receptacle which is partitioned into multiple fluidly isolated sections, each containing a region of the sputum sample, and wherein the reagent is added to a region contained within only a subset (e.g. one) of the sections. Optical sensing data can be acquired from all sections, allowing a true color of the sputum to be detected as well as the reagent color response. Different reagents could be added to different of the sections.

In accordance with one or more embodiments, the measurement protocol may further comprise: acquiring preliminary optical sensing data relating to the sputum sample both in advance of the conditioning procedure and subsequent to the conditioning procedure; determining a defined property of the sputum sample based on the data acquired before and after the conditioning procedure; and determining a success of the conditioning procedure based on a change in the property.

The property may for example be color homogeneity.

By way of example, if there is little detectable change in color homogeneity between the two measurements, this may indicate that the pre-conditioning step was unsuccessful, which could arise, for instance if insufficient conditioning agent (e.g. saline) was mixed with the sputum sample. This may then trigger an alarm, and/or additional pre-conditioning step.

In accordance with one or more embodiments, the measurement device may further comprise a user interface including a display device, and wherein the processing arrangement is adapted to communicate a display output indicative of the results of the analysis operation to the user interface for presentation on the display device.

Examples in accordance with a further aspect of the invention provide a processing arrangement comprising one or more processors and an input/output, the input output adapted to be connected to an optical sensing device during operation. The processing arrangement adapted to: receive at the input/output optical sensing data relating to a plurality of spatial locations of the sputum sample; determine based on the sensing data a respective sputum color measurement for each of the plurality of spatial locations; apply an analysis operation comprising determining one or more properties of the sputum sample and/or one or more health parameters of the subject, based on the color measurements for the plurality of spatial locations; and generate a data output based on the result of the analysis operation.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying schematic drawings, in which:
Fig. 1 outlines steps of an example analysis method according to one or more embodiments;
Fig. 2 shows an example sputum sample for analysis;
Fig. 3 shows an example measurement area of a sample including multiple measurement locations;
Fig. 4 shows a set of example color measurement results for different samples taken at different times;
Fig. 5 shows a set of example color measurement results for the same sample taken at different times over a measurement period;
Fig. 6 shows an example measurement device in accordance with one or more embodiments;
Figs. 7-8 show the example measurement device of Fig. 6 in operation during each of a sample conditioning step and an optical sensing step;
Figs. 9-10 show a further example measurement device according to one or more embodiments; and
Fig. 11 shows an example color measurement for an embodiment in which a reagent is added to a section of the sample to detect presence of an analyte.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for analysis of sputum samples of a subject, for use in monitoring health progression of the subject. The method comprises acquiring optical sensing data of a sputum sample at a plurality of spatial locations across the sample, and determining a respective color measurement for each of the spatial locations. The method further comprises determining a property associated with the sample, or determining health information related to the patient, based on the combination of color measurements from the plurality of locations. Thus, more detailed and precise analysis of the sputum can be achieved compared to acquiring a single color measurement, which allows for more precise monitoring of the patient progress. Additional information not attainable with a single color measurement can also be derived.

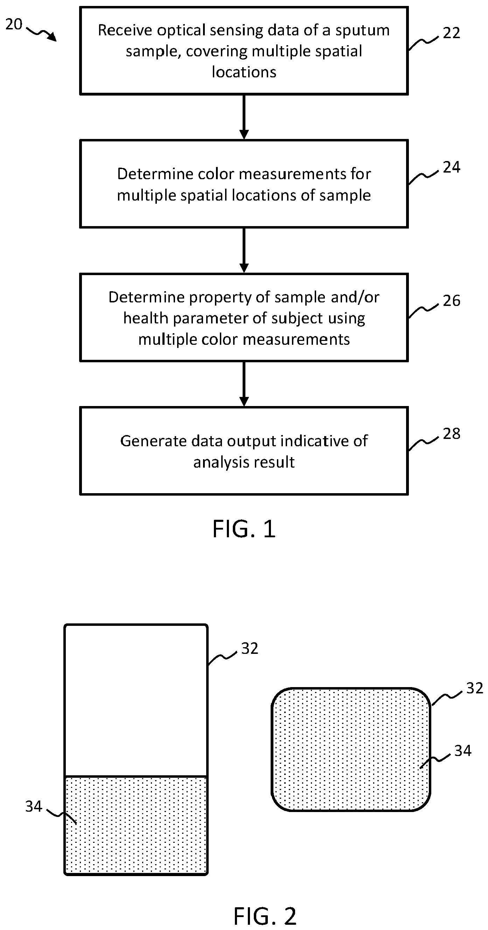

Fig. 1 outlines steps of an example method for analyzing a sputum (or mucus) sample of a subject according to one or more embodiments of the invention. The method 20 comprises receiving 22 from an optical sensing device optical sensing data relating to a plurality of spatial locations of the sputum sample. The optical sensing data may be image data in some examples, for example captured by a camera. Here, the different pixels of the resulting image may provide optical sensing data for the plurality of locations. In further examples, the optical sensing data may be spectrophotometer data. Any other type of optical measurement data may also be used, provided that the data carries color-dependent information, for instance spectral information or color image information. The optical sensing data should cover a plurality of spatial locations, for instance a plurality of locations across a particular cross-section of the sputum sample.

The method further comprises determining 24, based on the sensing data, a respective sputum color measurement for each of the plurality of spatial locations. This step may comprise ascribing a color value to each location. There may be a finite set of possible colors, e.g. 256, and wherein one of the colors is ascribed to each location based on the optical sensing data. A pre-determined algorithm may be applied to derive the explicit color measurements for each location based on the sensing data. The result of this step may for example be a data array listing color values for each of the plurality of spatial locations.

The method further comprises performing 26 an analysis operation comprising determining one or more properties of the sputum sample and/or one or more health parameters of the subject based on the color measurements for the plurality of spatial locations. The analysis operation may be implemented using a pre-stored or pre-determined algorithm or program. The analysis operation may comprise accessing or retrieving reference color information, e.g. stored in a local or remote datastore. Determining the one or more properties or parameters may comprise comparing the color measurement information with the reference information. The reference information may be a dataset which stores different possible sets of color information and associated properties of the sputum or different health parameters or indications for the subject. In some examples, an intermediate step may be performed of collating, pre-processing or combining the color measurements in advance of determining the property or parameter. For example, a step may be performed of identifying a list of all of the different colors present among the plurality of locations.

The method further comprises generating a data output based on the result of the analysis operation. The data output may be a display output for controlling a display unit to display results of the analysis operation. In further examples, it may simply be a data packet carrying the results of the analysis for communication to a further device or for storage in a datastore.

The method 20 is a computer-implemented method and may be performed for example by a processing arrangement comprising one or more processors. The processing arrangement may be adapted in operation to establish a communication channel with an optical sensing device for receiving the optical sensing data.

In operation, a user may provide a sample of sputum or mucus into a dedicated receptacle, and optical sensing is performed on the sample, to thereby acquire the optical sensing data for a plurality of spatial locations across the sample. The details of an example system for performing the sensing will be discussed in greater detail later in this disclosure.

Fig. 2 schematically illustrates an example sputum sample 34 collected in a sample receptacle 32. Fig. 2 (left) shows an elevation view of the sample 34 in the receptacle 32, and Fig. 2 (right) shows a plan view of the sample 34 in the receptacle 32. The optical sensing data may comprise optical sensing data for a plurality of spatial locations across a cross-section of the sample. For instance, an optical sensing device may in operation capture sensing data from a sensing position above the sample or below the sample.

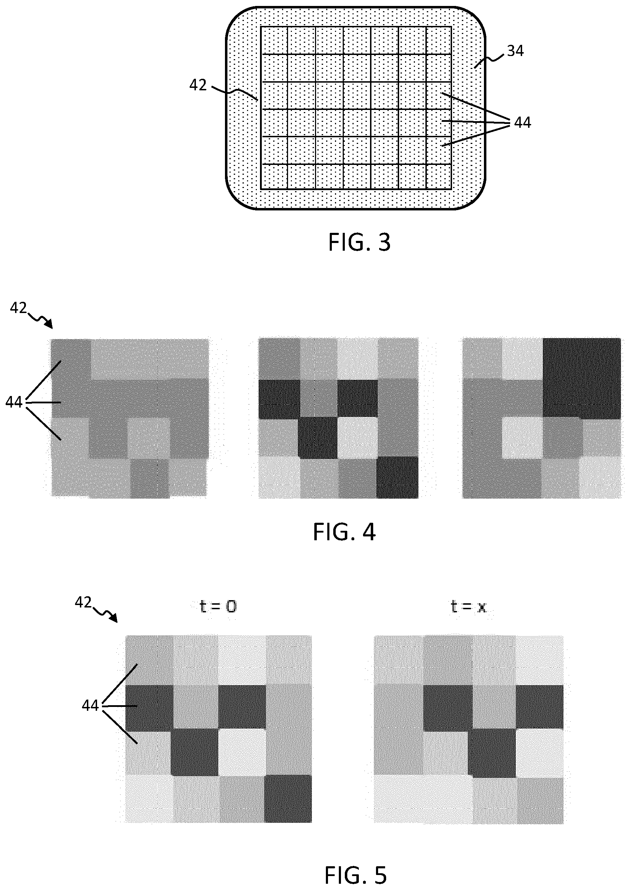

Fig. 3 schematically illustrates one example optical measurement area 42 across a sample 34, which is shown schematically divided into a plurality of measurement sub-areas 44 having different spatial locations. In this example, these define a grid. Optical sensing data are acquired for each of the spatial locations 44. The different spatial locations may for example correspond to pixel locations of a captured image. The measurement areas 42 may for example be a notional plane across a cross-section of the sample.

Due to the obtaining of color measurements for a plurality of spatial locations, high resolution analysis can be performed. By way of example, a spatial distribution of color can be detected and used to assess the properties of the sputum or derive information about the subject's health.

The one or more properties of the sputum 34 derived in the analysis operation may by way of example include: color homogeneity, spatial color distribution, color composition (e.g. list of all colors present), color spread (e.g. number of colors present). The one or more properties may additionally or alternatively include medically relevant properties such as bacterial load, or binary properties indicative of presence of absence of one or more pre-defined warning colors, e.g. red for blood.

In one set of examples, the analysis operation may comprise determining a measure indicative of color homogeneity of the sample. This could be determined for example using a pre-determined algorithm configured to map a set of input color data for a plurality of spatial locations (e.g. in array form) to a value of a homogeneity metric.

Homogeneity can be derived from the amount and distribution of colors across the plurality of spatial locations 44. If the number of different colors is lower, the sample is more homogeneous. In addition, if the distribution of the colors is more evenly spread over the sample (over the plurality of spatial locations), the sample is also more homogeneous.

Homogeneity is a clinically relevant property. In particular, the homogeneity may give an indication of whether a patient is adherent to medication, for example taking mucolytic medication. It may also provide an indication of disease progression and clinically important changes in a patient's condition. For example, when bacterial load increases, the number of different colors present in the sample increases. For example, new colors may appear, such as yellow and green. Furthermore, when infection occurs, blood may be present (color red). Dried blood appear as brown or black.

While a sputum sample is never completely homogeneous, a sputum sample of a healthy person is almost clear, although with some irregularities (mucin clusters that appear white). It is easily distinguished from a sample produced by an unhealthy person, which typically has a greater number of colors, and greater spatial irregularity in color.

In addition to, or instead of, deriving properties of the sputum sample, health information related to the subject can be determined based on the color measurement information. This may be based on use of reference color information which associates different sets of color measurements, or information derived therefrom, with different health conditions and/or different severities of health condition. The information derived may include for example, a medical condition of the subject, a severity of a medical condition, and/or an indicator of sputum removal efficacy.

In one set of examples, the analysis operation comprises detecting presence of any of a set of pre-defined reference colors at any of the spatial locations. The reference colors could be indicative of danger signs, for example blood presence.

Additionally or alternatively, further advantageous embodiments may comprise compiling a list or record of which of a set of pre-defined reference colors is detected among any of the plurality of locations, and wherein the particular combination of colors present in the sample across the plurality of locations is used to determine health information for the subject. For example, there may be stored a reference look-up-table (e.g. similar to Table 1 above) which records medical conditions associated with different combinations of sputum colors.

In accordance with some examples, additionally, an average or weighted average of the color measurement for the plurality of spatial locations may be derived. This provides an additional global representation of the sputum color. This can be a useful adjunct to the more detailed spatial color distribution information.

The higher resolution information derived from the plurality of color measurements allows for more nuanced and sophisticated tracking of patient progress over time based on sputum analysis.

In advantageous embodiments, the method may further comprise comparing results of the analysis operation with a source of historical sputum analysis information for the subject, and detecting change over time of one or more of the sputum properties or subject health parameters. For example, a trend in the one or more properties over a plurality of measurement events may be derived. Alternatively, simply a change in one or more of the properties from a previous measurement event may be detected.

The historical sputum analysis information means a dataset of previously derived values for at least one of the one or more properties of the sputum, or the one or more health parameters of the subject. Each instance of acquiring the color measurement information and deriving the sputum properties or health parameters may be termed a measurement event. The historical information stores the derived results of the analysis operation from previous measurement events. These may be time and date stamped. The historical analysis information may be retrieved from a local or remote datastore.

The method may further comprise appending the result of the analysis operation to a historical analysis dataset of the subject, along with a date and time stamp. For example the data output may be communicated to a local or remote datastore which stores a historical dataset for the subject.

The method may comprise generating an alert message if a derived change or trend exceeds a certain threshold for the gradient with respect to time.

For example, a subject may provide a sample at regular time intervals, for instance once each day, and color measurements acquired for each sample. Changes over time in properties of the samples can be monitored. The detected changes or trends may for example be changes in the color composition (i.e. the particular set of colors which are visible among the different spatial locations). This could be used to detect first appearance of blood in the sputum for example, where the color composition changes to include for the first time the color red. Such a change would normally not be detectable where just a single color measurement is acquired through a whole sample, since the small area of red would be averaged out among the rest of the colors of the sample. The detected changes may further include changes in color homogeneity, or changes in the spatial distribution of the colors, or changes in any other property (including for instance dynamic properties which will be discussed in more detail to follow).

Fig. 4 schematically illustrates changes in color measurement information for different sputum samples collected from a single patient at a series of three time points.

The trend data can be used to determine relevant health information to the subject, for example sputum removal efficacy and therapy effectiveness. Using disease-specific sputum color data as reference data, advice may be generated for presentation to a user based on the trend information, for example advice to seek medical assistance by a pulmonologist.

The multi-location color information the sample also provides the possibility of acquiring dynamic color information for a sample, based on capturing optical sensing data for a single sputum sample over an extended time window. In particular, the optical sensing data for the sample may include sensing data for a series of time points spanning a measurement period. The color measurements may be derived for the plurality of spatial locations for each of the series of time points.

A measure indicative of changes in a spatial color distribution across the sample over the measurement period may be determined. This is derived based on the changes in color distribution across the plurality of spatial locations.

A measure indicative of changes in a color homogeneity of the sample over the measurement period may be determined.

Monitoring changes in the amount and distribution of colors over a set measurement period also allows information about the stability of the sample to be determined, for example after pre-treatment of the sample.

Fig. 5 schematically illustrates change in the color measurement information for a single sample over a measurement period. Fig. 5 (left) shows the sample color measurements for multiple spatial locations 44 at a first time t=0, and Fig. 5 (right) shows the color measurement information at a later time point in the measurement period, t=x. Data for more than two time points would typically be acquired however.

Additionally or alternatively, in accordance with one or more examples, the analysis operation may comprise identifying one or more physical features within the sample based on the sensing data, and tracking movement of features over the measurement period.

The movement of such physical features may be due to Brownian motion and/or due to an external pressure flow exerted manually. By monitoring this, rheological (flow) behavior of the sample can be derived, and also the speed of the features. As an example, a high viscous sputum sample will have different flow characteristics to a low viscous sample. Viscosity has a clinical significance. For example, mucus with a higher viscosity tends to accumulate more in the airways. This may lead to exacerbations and increased risk of infection. Mucus with a low viscosity can easily be transported out of the airways (and can be swallowed or expelled).

Based on the one or more derived properties of the sputum, or the derived health information for the subject, the method may further comprise generating user advice or guidance for prompting a health management action by the user. This may comprise generating an alert, notification, or information message for informing the subject or caregiver about the derived health status of the subject, or of a recommended action to be taken, such as scheduling a clinical appointment with a clinician.

In accordance with one or more embodiments, the method may further comprise a control procedure, performed in advance of the data processing procedure, the control procedure comprising controlling an optical sensing device to acquire optical sensing data of a sputum sample. For example, the method may be performed by a processing arrangement and wherein the processing arrangement is operatively coupled with a measurement device comprises an optical sensing means for acquiring the optical sensing data.

Examples in accordance with a further aspect of the invention provide a system for acquiring the optical sensing data of the sample and executing the analysis method based on that data. There are different options for the implementation of the system. In all cases, an aim is that the system permits measurement data to be acquired easily and reliably in a home environment. In particular, it is preferable that the system is operator independent, so that the measurement conditions are as consistent as possible for every measurement.

The general features of the system are as follows. The system comprises a measurement device, wherein the measurement device comprises: a housing, and an optical sensing device fixed relative to the housing and operable to emit optical signals into a sensing region and acquire optical sensing data based thereon, for thereby acquiring optical sensing data of a sputum sample received in the sensing region. The system further comprises a processing arrangement adapted to receive optical sensing data from the optical sensing device and perform an analysis method in accordance with any example or embodiment outlined above or described below, or in accordance with any claim of this application. The processing arrangement may be integrated in the measurement device, or external to it.

There are two main options for implementing a measurement device for acquiring the optical sensing data. One is use of a tabletop device with integrated optical sensing apparatus, and the other is use of handheld device having an optical sensing apparatus. The latter option may be smaller in size, less complex in design, and easier to operate. However, the tabletop device may allow for more controlled measurement conditions, particularly relating to the physical conditions of the sample, and the optical arrangement of the sensing device relative to the sample. This may lead to measurement results with greater reproducibility.

If reproducibility is a priority, an integrated table-top measurement device may be preferred. The device may comprise a display and a user interface. It may be mains powered. It may employ use of an optical sensing device which utilizes optical reflection, optical transmission, or both. This type of device may be most suitable for use where multiple measurements are needed at very regular intervals, for example a patient recovering at home, where color samples are needed multiple times per day.

If less regular measurements are needed, or if mobility is a priority, the use of a handheld measurement device may be preferable. The costs for this type of device may be lower. The handheld device may be a dedicated handheld device with an integrated optical sensor, e.g. a spectrophotometer. In other examples, it may for instance be facilitated by a mobile computing device, such as a smartphone. The mobile computing device may include an integrated camera which can be used to perform an optical reflectance measurement of the sample. An app may be installed on the device, adapted to control operation of the camera to acquire the reflectance measurement data. The analysis method for analyzing the optical sensing data may be performed by a processing arrangement comprised by the handheld device itself, for example by an app installed on the mobile computing device. Alternatively, the analysis may be done externally, e.g. using a remote computer or a cloud-based processing arrangement.

One illustrative use case for a handheld type measurement device may be for a patient suffering bronchiectasis, and using a High Frequency Chest Wall Oscillation (HFCWO) vest each morning. A sputum sample may be measured after each therapy session. Data may be directly transmitted to an external computer at a hospital site, for review by a pulmonologist.

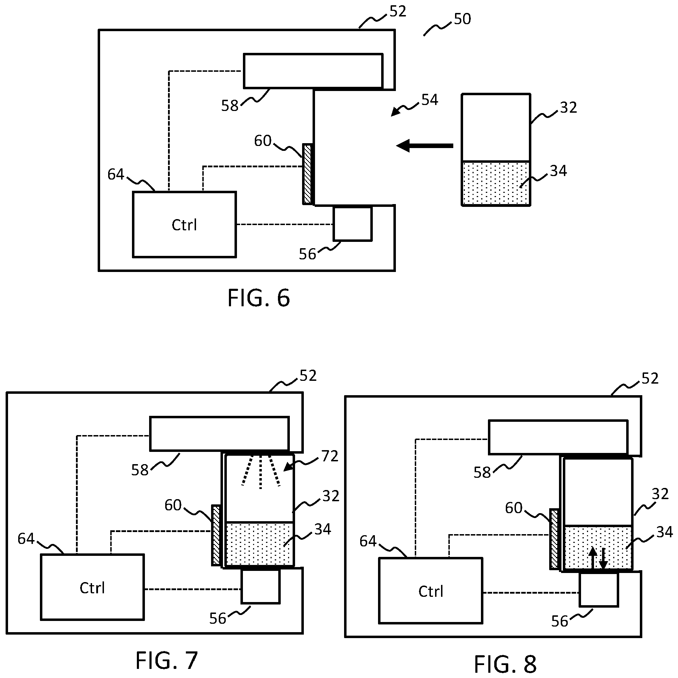

One example measurement device according to one or more embodiments of the sputum analysis system is shown in Figs. 6-8. One aspect of the invention provides the measurement device 50 by itself. Another aspect provides the measurement device as part of a system, further comprising a processing arrangement (not shown in Fig. 6) adapted to perform the analysis method.

The measurement device 50 comprises a housing 52. The housing delimits a receptacle receiving section 54 arranged to receive a removable sputum sample receptacle 32, and adapted to hold the receptacle within a sensing region of an optical sensing device 56 during operation, for optical sensing of a sputum sample 34 in the receptacle. The receiving section 54 may be shaped to co-operatively receive and hold a sample receptacle of a specific shape and dimensions. This ensures that only a receptacle of a particular defined geometry can be received in the measurement device, so that this geometry is uniform for all measurements.

The measurement device 50 is adapted to receive and hold the sample receptacle in a defined position relative to the optical sensing device 56. This provides for more reliable and reproducible results. The measurement device may in this case be a tabletop measurement device for instance. However, this is not essential.

In order to ensure the measurements are as reproducible as possible, it is preferable that the measurement device 50 includes means for controlling measurement conditions. In the example of Figs. 6-8, the measurement device comprises a heating assembly 60 comprising one or more heating elements (e.g. Peltier elements), arranged for controlling a temperature of the sputum sample 34 in the sputum receptacle when received in the receiving section 54. The one or more heating elements of the heating assembly 60 are arranged to be in thermal communication with the sample receptacle and/or sample contents when the receptacle is received in the receiving section 54. A control module 64 is adapted to control a temperature of the sample 34 to be within a pre-defined temperature range using the heating assembly. The heating assembly may further include a temperature sensor, such as a thermistor, for use in controlling the temperature of the sample. By controlling the temperature, a viscosity of the sample may be controlled for example.

In order to perform measurements and acquire the optical sensing data of the sample 34, the measurement device 50 comprises a control module 64 adapted to execute a pre-defined measurement protocol. The measurement protocol comprises receiving a signal indicative of receipt of the sputum sample 32 in the receiving section 54. For example, a presence sensor may be included in the receiving section adapted to sense when the receptacle is fitted in place. The presence sensor may generate the signal.

Responsive to receipt of the signal, the control module 64 activates execution of a sample conditioning procedure. The sample conditioning procedure is for adjusting one or more physical properties of the sample, for ensuring consistency in conditions between measurements. The physical properties may include for example temperature, consistency, viscosity, composition, optical transmissivity or any other property.

The measurement protocol further comprises, subsequent to the conditioning step, activating an optical sensing procedure to acquire the optical sensing data of the sample 34. This comprises controlling the optical sensing device 56 to acquire optical sensing data.

The control module 64 may be a part of the same processing arrangement (which performs the analysis procedure) or may be separate to it.

One part of the conditioning procedure may be controlling the temperature of the sample using the heating assembly 60, as discussed above.

A further or alternative part of the conditioning procedure may comprise adding a sample conditioning agent into the sample. It preferably further comprises mixing the conditioning agent into the sample. The conditioning agent may be an agent which has the property of altering a consistency or viscosity of the sputum sample. For example it may include a saline solution. The conditioning agent does not change a color of the sample.

Fig. 7 schematically illustrates the conditioning procedure stage of the measurement protocol. In this example, the conditioning procedure comprises adding a conditioning agent 72 to the sample, for example a saline solution.

For this purpose, the measurement device 50 comprises a conditioning agent dispensing unit 58. This may include a conditioning agent reservoir and a dispensing nozzle positioned to permit dispensing of the conditioning agent 72 into the sputum sample 34 in the receptacle 32 when the receptacle is received in the receiving section 54. In this example, the conditioning agent dispensing unit is positioned above the receiving section, so that the conditioning agent is dropped down into the sample 34 from above.

Actuation of the conditioning agent release may be automated and controlled by the control module 64. Alternatively, it may be manually actuated. For example, the measurement device 50 may include a push mechanism arranged such that insertion of the sample receptacle 34 in the receiving section 54 actuates, e.g. through hydraulic pressure, dispensing of a set volume of the conditioning agent.

The measurement device 50 may further comprise a mixing assembly (not shown) adapted to permit mixing of the sputum sample when the sample receptacle is received in the receiving section. For example this may be a stirring shaft arranged for being inserted into the sample receptacle during operation, or a magnetic stirring assembly, or a shaking or agitating assembly arranged to shake or agitate the sample.

Fig. 8 schematically illustrates the optical sensing procedure.

The optical sensing device 56 may be any type of optical sensing device capable of acquiring color or spectral information. In some examples, it may comprise an RGB camera. In further examples, it may comprise a spectrophotometer. The optical sensing device may use optical transmission sensing, or optical reflection sensing. In the former case, an optical generator and optical sensor would need to be provided facing one another on opposite sides of the sample receptacle 32. In the example illustrated in Fig. 8, optical reflectance is used. A light source and an optical detector are integrated in the same unit 56.

In accordance with one or more embodiments, the measurement protocol may further comprise a procedure for checking success of the sample conditioning procedure. This comprises acquiring preliminary optical sensing data relating to the sputum sample 34 both in advance of the conditioning procedure and subsequent to the conditioning procedure. A defined property of the sputum sample is determined based on the data acquired before and after the conditioning procedure. A success of the conditioning procedure is then determined based on detecting a change in said property.

The property may for example be color homogeneity of the sample. By way of example, if there is little detectable change in color homogeneity between the two measurements, this may indicate that the pre-conditioning step was unsuccessful, which could arise, for instance if insufficient conditioning agent 72 (e.g. saline) was mixed with the sputum sample. This may then trigger an alarm, and/or an additional pre-conditioning step. Depending upon the starting conditions of the sample, different amounts of the conditioning agent may be needed in order to achieve the target measurement condition (i.e. the target value for the detected property). For example, the success of the conditioning step may be determined based on the change in the property falling within a pre-defined target range, or exceeding a target threshold.

As discussed, an advantage of use of a measurement device 50 having an integrated optical sensing device 56 and sample receptacle holding section 54 is that measurement conditions can be better controlled. To further control conditions, it is preferable that the external conditions associated with the measurement are also controlled. These may include for example any of: the structure and geometry of the sample receptacle 32, the protocol followed by the user in preparing the sample and filling the receptacle, the timings at which measurements are performed, clearance of mouth contamination (e.g. mouth rinsing before taking each sample), and storage or conditioning of the sample between taking of the sample and the performing of the measurement.

With regards to the sample receptacle 32, it is preferable that this is disposable (to prevent cross-contamination between samples), with pre-defined dimensions and internal volume (to aid consistency of the sample volumes and consistency in optical conditions when acquiring optical data). It preferably includes markings to indicate a target fill level to the user. This helps ensure a consistent volume of sputum is used for every measurement. The receptacle is preferably made of optical grade plastic, and/or includes an optical window formed of optical grade plastic (e.g. Cyclo Olefin Polymer), the optical window arranged to be aligned optically with the optical path of the optical sensing device 56 when the receptacle is received in the receiving section 54.

For optical measurements, the optical path between the light source and detector of the optical sensing device 56 is important. This is the case for both transmission and reflectance measurements. An aim is to minimize measurement noise. Sources of noise include poor alignment of the sample with the optical sensor, contamination of the sample, and drift in time (within and between samples). By using disposable parts of optical grade material, it is possible to mitigate the first two factors. Cleaning of the system between measurements is also not needed. With regards to drift in time, stabilization of the sputum sample may be established using the sample conditioning procedure, which may include for example controlling the temperature or adding a conditioning agent, to control viscosity. The sample conditioning procedure may further include a waiting (or settling) time of pre-determined length to allow the sputum sample 34 to reach a steady state in temperature and to physically settle.

One aspect of the invention may provide a kit of parts comprising a measurement system according to any of the embodiments described in this disclosure, in combination with at least one sputum sample receptacle 32 with one or more of the features mentioned above.

A workflow of a typical measurement event may follow an ordered set of steps, some performed by a user, some implemented automatically by the analysis system. By way of non-limiting illustration, a typical workflow of a measurement event might follow a set of steps as follows:
Remove sample receptacle 32 from a blister pack in which it is stored;
Fill receptacle 32 with sputum sample (using min and max level markings as a guide);
Place receptacle 32 in the measurement device 50;
   Sample conditioning procedure is triggered, through manual actuation upon inserting receptacle, or automatically upon inserting receptacle, or through a user-interface control;
The conditioning procedure is subsequently triggered, and proceeds for a fixed time;
Following conditioning, the optical measurement starts automatically;
The analysis operation is applied to the acquired optical sensing data;
The system displays results of the analysis operation to a user via a display; The user removes and disposes of the sample receptacle.

In accordance with one or more embodiments, the measurement device 50 may further comprise a user interface including a display device, and wherein the processing arrangement is adapted to communicate a display output indicative of the results of the analysis operation to the user interface for presentation on the display device.

In advantageous examples, the control module 64 of the measurement device may be further adapted to control the display unit to display operation instructions, for use by a user. By displaying the instructions, this aids reliable and consistent implementation of each measurement, e.g. filling the sputum sample receptacle to the correct level, fitting the sample receptacle in the measurement device correctly, and properly removing and disposing of the sample receptacle after completion.

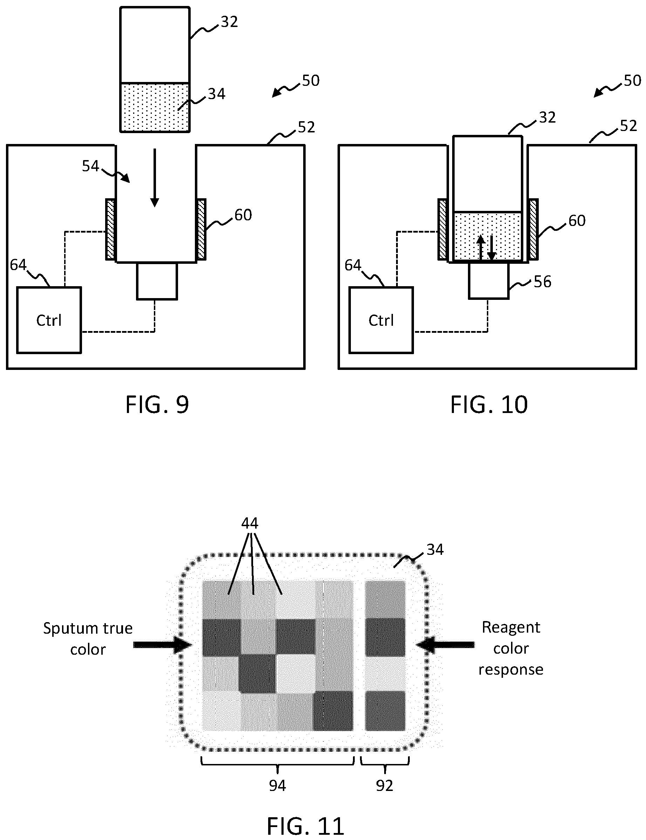

For purposes of further illustration, Figs. 9-10 show one further example measurement device 50 according to one or more embodiments. The features of this example are substantially the same as those of the example of Figs. 6-8, except that the sample receptacle 32 is loaded vertically into a sample receiving section 54, and there is no dispensing unit 58 for dispensing a conditioning agent. By way of example, the conditioning agent may be already be present in the sample receptacle 32, for instance as a coating on the walls of the receptacle, or added manually by a user. Alternatively, no conditioning agent may be added. The measurement device 50 includes the heating assembly 60 for controlling the temperature of the sample.

In example measurement devices 50 outlined above, the optical measurement data for the sample 34 is acquired with the sample contained in the sample receptacle 32. However, in variations on these example measurement devices, the device may further include a measurement chamber, separate from the sample receptacle. The device may be adapted to fluidly transport a fixed volume of the sample 34 from the sample receptacle into the measurement chamber. The optical sensing data may then be acquired for the sample with the sample received in the measurement chamber. In this case, the optical sensing device 56 is moved so that it is in optical communication with the measurement chamber. Additional fluid pumping components may be added for transferring the sample from the received sample receptacle to the measurement chamber.

The measurement chamber may be removable, and preferably disposable, to further prevent contamination between samples. The measurement chamber may have a fixed volume, smaller than that of the sample receptacle 32, to ensure that the optical sensing data is acquired for a sample with fixed volume, even if the receptacle is overfilled or underfilled. In some examples, some or all of the steps of the sample conditioning procedure (if one is performed) may be performed with the sample received in the measurement chamber. For example, the temperature control may be performed with respect to the sample in the measurement chamber, instead of, or in addition to, when the sample is in the sample receptacle. In some examples, the addition of the sample conditioning agent (if this is used), and optional mixing step, may be performed with the sample in the sample receptacle.

All other options and features described above in relation to the measurement device 50 may be applied also to this variant of the measurement device having the separate measurement chamber.

In accordance with one or more embodiments, the measurement protocol may further comprise adding a reagent to at least one region of the sample, the reagent adapted to stimulate a color-change response dependent upon an analyte content in the sample. The measurement protocol may further comprise acquiring optical sensing data of said at least region for use in detecting the color-change response.

A wide variety of different analyte parameters may be detected using color indicators, such as pH, electrolytes, lipids, and proteins (e.g. immunoglobulins, or glycoproteins such as mucins).

Detection of presence and concentration levels of an analyte is possible.

The optical sensing operation should comprise acquiring optical sensing data of the reagent color change response in addition to detecting the true, original color of the sputum sample. This can be achieved in different ways. One way is to use a sample receptacle 32 which is partitioned into multiple fluidly isolated sections, each containing a region of the sputum sample, and wherein the reagent is added to a region contained within only a subset (e.g. one) of the sections. Optical sensing data can be acquired from all sections, allowing a true color of the sputum to be detected as well as the reagent color response. Different reagents could be added to different sections.

Another option may be to add the reagent only after acquiring a first set of optical sensing data of the sputum sample, and subsequently acquire a second set of optical sensing data representative of the color change response to the reagent.

The color change response can be compared with reference data stored on a local or remote datastore to determine one or more properties of the sample, or health parameters of the subject.

The measurement device may comprise a reagent dispensing unit operable to dispense one or more different reagents into the sputum sample in the receptacle when the receptacle is received in the receiving section. It may include a reagent reservoir or storage chamber and a dispensing outlet or nozzle arranged to dispense the reagent to the sample when the receptacle is in the receiving section.

The receptacle may comprise one or more partitions, dividing the receptacle into multiple fluidly isolated sections, each for containing a region of the sputum sample.

Fig. 11 schematically illustrates example color measurement data for a sample to which a reagent has been added in one region 92 of the sample receptacle, said region being fluidly isolated from the remainder of the volume of the receptacle, the remainder of the volume forming a second region 94. Optical sensing data are acquired for a plurality of spatial locations 44 in each of the first 92 and second 94 regions, and color measurements are obtained for each of the plurality of spatial locations in each region.

Examples in accordance with a further aspect of the invention provide a processing arrangement comprising one or more processors and an input/output, the input output adapted to be connected to an optical sensing device during operation. The processing arrangement adapted to: receive at the input/output optical sensing data relating to a plurality of spatial locations of the sputum sample; determine based on the sensing data a respective sputum color measurement for each of the plurality of spatial locations; apply an analysis operation configured to determine one or more properties of the sputum sample and/or one or more health parameters of the subject, based on the color measurements for the plurality of spatial locations; and generate a data output based on the result of the analysis operation.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A sputum analysis method (20) for analyzing a sputum sample (34) of a subject, the method comprising:
receiving (22) from an optical sensing device (56) optical sensing data relating to a plurality of spatial locations (44) of the sputum sample;
determining (24) based on the sensing data a respective sputum color measurement for each of the plurality of spatial locations;
performing (26) an analysis operation comprising determining one or more properties of the sputum sample and/or one or more health parameters of the subject, based on the color measurements for the plurality of spatial locations; and
generating (28) a data output based on the result of the analysis operation.

2. A method (20) as claimed in claim 1, wherein the analysis operation comprises determining a measure of color homogeneity of the sample (34) based on the color measurements for the plurality of spatial locations (44).

3. A method (20) as claimed in claim 1 or 2, wherein the analysis operation comprises detecting presence of any of a set of pre-defined reference colors at any of the spatial locations (44).

4. A method (20) as claimed in any of claims 1-3, wherein the method further comprises comparing results of the analysis operation with a source of historical sputum analysis information for the subject, and detecting a change over time of one or more of the derived sputum properties or health parameters.

5. A method (20) as claimed in any of claims 1-4, wherein the optical sensing data for the sample (34) includes optical sensing data for a series of time points spanning a measurement period, and wherein the color measurements are derived for the plurality of spatial locations for each of the series of time points.

6. A method (20) as claimed in claim 5, wherein
the analysis operation comprises determining a measure indicative of changes in a spatial color distribution across the sample (34) over the measurement period; and/or
the analysis operation comprises identifying one or more physical features within the sample based on the sensing data, and tracking movement of the features over the measurement period.

7. A computer program product comprising computer program code configured, when run on a processor, to cause the processor to perform a method in accordance with any of claims 1-6.

8. A sputum analysis system, comprising:
a measurement device (50), the measurement device comprising:
a housing (52), and
an optical sensing device (56) fixed relative to the housing and operable to emit optical signals into a sensing region and acquire optical sensing data based thereon, for thereby acquiring optical sensing data of a sputum sample (34) received in the sensing region; and
a processing arrangement adapted to receive optical sensing data from the optical sensing device and perform an analysis method according to any of claims 1-7.

9. A system as claimed in claim 8, wherein the measurement device (50) further includes a receptacle receiving section (54) arranged to receive a removable sputum sample receptacle (32), and preferably adapted to hold the receptacle within said sensing region during operation for optical sensing of a sputum sample (34) in the receptacle.

10. A system as claimed in claim 8 or 9, wherein
the processing arrangement is integrated in the measurement device (50); or
the processing arrangement is comprised by a mobile computing device, and adapted to establish a communication channel with the measurement device during operation to receive the optical sensing data.

11. A system as claimed in claim 9 or 10, wherein the measurement device comprises a control module (64) adapted to execute a pre-defined measurement protocol, the measurement protocol comprising:
receiving a signal indicative of receipt of the sputum sample in the receiving section (54);
executing a sample conditioning procedure responsive to receipt of the signal, for adjusting one or more physical properties of the sputum sample (34); and
subsequently executing an optical sensing procedure to acquire the optical sensing data of the sample.

12. A system as claimed in claim 11, wherein the conditioning procedure comprises:
adjusting a temperature of the sample to within a pre-defined temperature range based on use of one or more heating elements (60) comprised by the measurement device; and/or
adding a sample conditioning agent (72) into the sample.

13. A system as claimed in any of claims 11-12, wherein the measurement protocol further comprises:
adding a reagent to at least one region (92) of the sample (34), the reagent adapted to stimulate a color-change response dependent upon an analyte content in the sample; and
acquiring optical sensing data of said at least region for use in detecting the color-change response.

14. A system as claimed in any of claims 11-13, wherein the measurement protocol further comprises:
acquiring preliminary optical sensing data relating to the sputum sample both in advance of the conditioning procedure and subsequent to the conditioning procedure;
determining a defined property of the sputum sample based on the data acquired before and after the conditioning procedure; and
determining a success of the conditioning procedure based on a change in the defined property.

15. A processing arrangement comprising one or more processors and an input/output, the input/output adapted to be connected to an optical sensing device during operation, and the processing arrangement adapted to:
receive at the input/output optical sensing data relating to a plurality of spatial locations (44) of a sputum sample (34) of a subject;
determine based on the optical sensing data a respective sputum color measurement for each of the plurality of spatial locations;
apply an analysis operation comprising determining one or more properties of the sputum sample and/or one or more health parameters of the subject, based on the color measurements for the plurality of spatial locations; and
generate a data output based on the result of the analysis operation.
